# EUROPEAN PATENT APPLICATION

(11) **EP 3 043 158 A1**
(43) Date of publication of application: **13.07.2016**
(21) Application number: 14841706.6
(22) Date of filing: 03.09.2014
(51) Int. Cl.: G01F 25/00, G01F 1/00, A61M 16/01

(54) **FLOW CALIBRATION METHOD AND SYSTEM FOR AN ANESTHESIA MACHINE FLOW SENSOR**

(30) Priority: 03.09.2013 CN 201310395676
(71) Applicant: Beijing Aeonmed Co., Ltd., Beijing 100070 (CN)
(72) Inventor: HUA, Wei, Beijing 100070 (CN)
(74) Representative: Hoarton, Lloyd Douglas Charles
(86) International application number: PCT/CN2014/085839
(87) International publication number: WO 2015/032325

(57) **Abstract**

A flow calibration method and system for an anesthesia machine flow sensor, the method comprising the following steps: gradually increasing the flow rates of a plurality of anesthesia machines, and when preset conditions are satisfied, acquiring the voltage values of the flow sensors (S101); calculating the average voltage value of the plurality of sampling points of each anesthesia machine, and obtaining, by means of the average voltage value of the plurality of sampling points of each anesthesia machine, the default voltage curve of the flow sensors (S102); dividing the default voltage curve into 3 sections, and selecting in the 3 sections 4 calibration points and acquiring the voltage value of each calibration point (S103); calculating the voltage difference, for each calibration point, between the acquired voltage and the default voltage curve, and according to the voltage difference, accurately calibrating the flow of the anesthesia machine (S104). According to the method, by means of determining the calibration points within a segmented range and according to the voltage differences between the default voltage curve and the calibration points, the flows of other acquisition points can be determined, reducing the level of complexity of the calibration process and also ensuring calibration accuracy.

## Description

The present patent application claims the priority of the Chinese Patent Application No. 201310395676.0, entitled "Flow Calibration Method and System for Flow Sensors of Anesthesia Machines", filed on September 3, 2013 by BEIJING AEONMED CO., LTD., the disclosure of which is incorporated herein by reference in its entirety.

### TECHNICAL FIELD

The present invention relates to the technical field of anesthesia machine, and particularly to a flow calibration method and system for flow sensors of anesthesia machines.

### BACKGROUND ART

A flow sensor is a key component for monitoring inspiration flow and expiration flow in an anesthesia machine. The anesthesia machine during ventilation is capable of measuring real-time flow rates of inspiration and expiration, and calculating tidal volumes of the inspiration and expiration according to the flow rates of inspiration and expiration. Therefore, the degree of accuracy of the flow sensor is of very high importance.

It is needed to perform a calibration on a flow sensor before use in order to ensure a measuring precision of the flow sensor, that is, a corresponding data relationship table is established by measuring a sampling voltage of the flow sensor and a corresponding flow rate value. An actual flow value is calculated through the sampling voltage by looking up the table during the actual use.

A multi-point calibration method is the current calibration method for the flow sensor, that is, the flow rate value is increased gradually from zero point, and corresponding values of sampling voltages are recorded. Since it is needed to record a large number of acquired points in every calibration and the number of the data of the calibration reaches dozens, it has disadvantages of long consumption time, inconvenient operation and lower efficiency etc.

### SUMMARY OF THE INVENTION

The objectives of the present invention are to solve at least one of the above described technical defects.

To this end, one objective of the present invention is to provide a flow calibration method for flow sensors of anesthesia machines with the advantages of short consumption time and convenient operation.

Another objective of the present invention is to provide a flow calibration system for flow sensors of anesthesia machines.

In order to achieve the above-described objectives, an embodiment of one aspect of the present invention provides a flow calibration method for flow sensors of anesthesia machines comprising the following steps: gradually incrementing flow rates of a plurality of anesthesia machines, and acquiring voltage values of the flow sensors when a preset condition is satisfied; calculating an average voltage value of each anesthesia machine at a plurality of sampling points and obtaining default voltage curves of the flow sensors through the average voltage value of each anesthesia machine at the plurality of sampling points; dividing the default voltage curves into N sections, selecting N+1 calibration points in the N sections, and acquiring a voltage value of each calibration point, wherein the N is a positive integer greater than 1; and calculating a voltage difference value between the acquired voltage value of each calibration point and the default voltage curves, and accurately calibrating flows of the anesthesia machines according to the voltage difference values.

According to a method of an embodiment of the present invention, the calibration points are determined within sectioned intervals and the flows of other acquired points are determined according to the voltage difference values between the default voltage curves and the voltage of calibration points, therefore the degree of complexity of a calibration process is reduced, and it is convenient for a user to use, meanwhile the accuracy of calibration is ensured.

In one embodiment of the present invention, the N sections are three sections, and a first section is from a point that the flow rate is 0 L/min to a point that the flow rate is 5 L/min, a second section is from a point that the flow rate is 5 L/min to a point that the flow rate is 20 L/min, and a third section is from a point that the flow rate is 20 L/min to a point that the flow rate is 130 L/min.

In one embodiment of the present invention, the preset condition is that the flow rate of the anesthesia machines reaches an integral multiple of a preset flow rate.

In one embodiment of the present invention, the preset flow rates in the three sections are different from each other.

In one embodiment of the present invention, the N+1 calibration points are four calibration points, which are respectively a point that the flow rate is 0, a point that the flow rate is 3 L/min, a point that the flow rate is 12 L/min, and a point that the flow rate is 30 L/min.

In order to achieve the above-described objectives, another aspect of an embodiment of the present invention provides a flow calibration system for flow sensors of anesthesia machines comprising: an acquiring module configured for gradually incrementing flow rates of a plurality of anesthesia machines, and acquiring voltage values of the flow sensors when a preset condition is satisfied; a calculating module configured for calculating an average voltage value of each anesthesia machine at a plurality of sampling points and obtaining default voltage curves of the flow sensors through the average voltage value of each anesthesia machine at the plurality of sampling points; a sectioning module configured for dividing the default voltage curves into N sections, selecting N+1 calibration points in the N sections, and acquiring a voltage value of each calibration point, wherein the N is a positive integer greater than 1; and a calibrating module configured for calculating a voltage difference value between the acquired voltage value of each calibration point and the default voltage curves, and accurately calibrating flows of the anesthesia machines according to the voltage difference values.

According to a system of the embodiment of the present invention, the calibration points are determined within sectioned intervals and the flows of other acquired points are determined according to voltage difference values between default voltage curves and the voltage of the calibration points, therefore the degree of complexity of a calibration process is reduced, and it is convenient for a user to use, meanwhile the accuracy of calibration is ensured.

In one embodiment of the present invention, the N sections are three sections, and a first section is from a point that the flow rate is 0 L/min to a point that the flow rate is 5 L/min, a second section is from a point that the flow rate is 5 L/min to a point that the flow rate is 20 L/min, and a third section is from a point that the flow rate is 20 L/min to a point that the flow rate is 130 L/min.

In one embodiment of the present invention, the preset condition is that the flow rate of the anesthesia machines reaches an integral multiple of a preset flow rate.

In one embodiment of the present invention, the preset flow rates in the three sections are different from each other.

In one embodiment of the present invention, the N+1 calibration points are four calibration points, which are respectively a point that the flow rate is 0, a point that the flow rate is 3 L/min, a point that the flow rate is 12 L/min, and a point that the flow rate is 30 L/min.

Part of additional aspects and advantages of the present invention will be given in the following description, and part of additional aspects and advantages of the present invention will become obvious from the following description or comprehended by a practice of the present invention.

### BRIEF DESCRIPTION OF THE DRAWINGS

The above and/or additional aspects and advantages of the present invention will become obvious and easy to understand from the description of embodiments in conjunction with the following drawings, in which:
Fig. 1 is a flow chart of a flow calibration method for flow sensors of anesthesia machines according to one embodiment of the present invention;
Fig. 2 is a flow chart of calibrating a default voltage curve according to one embodiment of the present invention;
Fig. 3 is a graph of a flow rate and a voltage of a flow sensor according to one embodiment of the present invention;
Fig. 4 is a flow chart of calibrating a flow of a flow sensor of each anesthesia machine according to one embodiment of the present invention; and
Fig. 5 is a structural block diagram of a flow calibration system for flow sensors of anesthesia machines according to one embodiment of the present invention.

### DETAILED DESCRIPTION OF THE EMBODIMENT

Embodiments of the present invention are described in detail below, examples of which are shown in the drawings, wherein the same or similar reference numbers represent the same or similar elements or the elements of the same or similar functions throughout the disclosure. The following embodiments described with reference to the drawings are exemplary, only for explaining the present invention rather than limiting the present invention.

In the description of the present invention, it is understood that, orientation or position relationships indicated by terms "center", "longitudinal", "transversal", "upper", "lower", "front", "back", "left", "right", "vertical", "horizontal", "top", "bottom", "inner", "outer", etc. are based on the orientation or position relationships as shown in the drawings, merely for ease of the description of the present invention and simplifying the description only, rather than indicating or implying that the indicated apparatus or element must have a particular orientation or be constructed and operated in a particular orientation. Therefore, these terms should not to be understood as a limiting of the present invention. In addition, terms "first", "second" are merely for a descriptive purpose, and are not to be understood to indicate or imply a relative importance.

In the description of the present invention, it should be understood that, unless exactly specified and defined otherwise, terms "install", "connected with", "connected to" should be comprehended in a broad sense. For example, these terms may be comprehended as being fixedly connected, detachably connected or integrally connected; mechanically connected or electrically connected; or directly connected or indirected connected through an intermediate medium, or in an internal communication between the two elements. The specific meanings about the above described terms in the present invention may be understood for those skilled in the art according to specific circumstances.

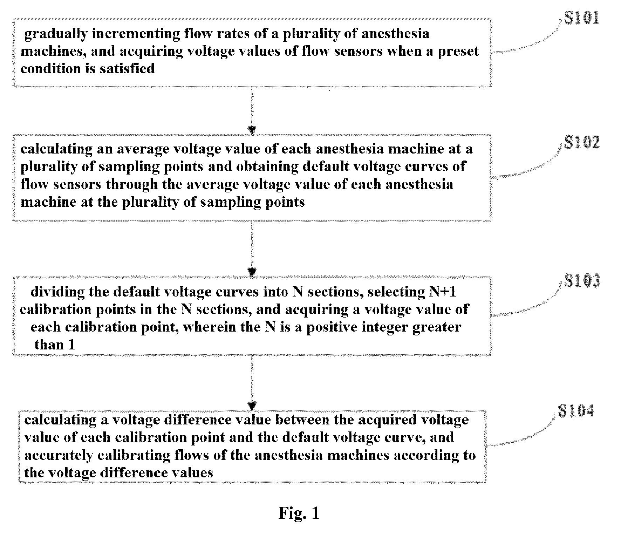

Fig. 1 is a flow chart of a flow calibration method for flow sensors of anesthesia machines according to one embodiment of the present invention. As shown in Fig. 1, the flow calibration method for flow sensors of anesthesia machines according to one embodiment of the present invention comprises the following steps:
Step 101, gradually incrementing flow rates of a plurality of anesthesia machines, and acquiring voltage values of flow sensors when a preset condition is satisfied.

In one embodiment of the present invention, the preset condition is that the flow rate of the anesthesia machines reaches an integral multiple of a preset flow rate. Preset flow rates in three sections are different from each other, the preset flow rate in a first section is 0.5 L/min, the preset flow rate in a second section is 1 L/min, the preset flow rate in a third section is 10 L/min.

Step 102, calculating an average voltage value of each anesthesia machine at a plurality of sampling points and obtaining default voltage curves of flow sensors through the average voltage value of each anesthesia machine at a plurality of sampling points.

Step 103, dividing the default voltage curve into N sections, selecting N+1 calibration points in the N sections, and acquiring a voltage value of each calibration point, wherein N is a positive integer greater than 1.

In one embodiment of the present invention, the N sections are three sections, and the first section is from a point that the flow rate is 0 L/min to a point that the flow rate is 5 L/min, and the second section is from a point that the flow rate is 5 L/min to a point that the flow rate is 20 L/min, and the third section is from a point that the flow rate is 20 L/min to a point that the flow rate is 130 L/min.

In one embodiment of the present invention, N+1 calibration points are four calibration points, which respectively are a point that the flow rate is 0, a point that the flow rate is 3 L/min, a point that the flow rate is 12 L/min and a point that the flow rate is 30 L/min.

Step 104, calculating a voltage difference value between an acquired voltage value of each calibration point and the default voltage curves, and calibrating accurately flows of the anesthesia machines according to the voltage difference values.

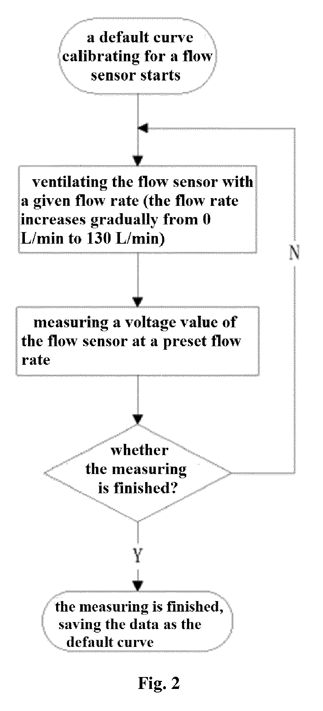

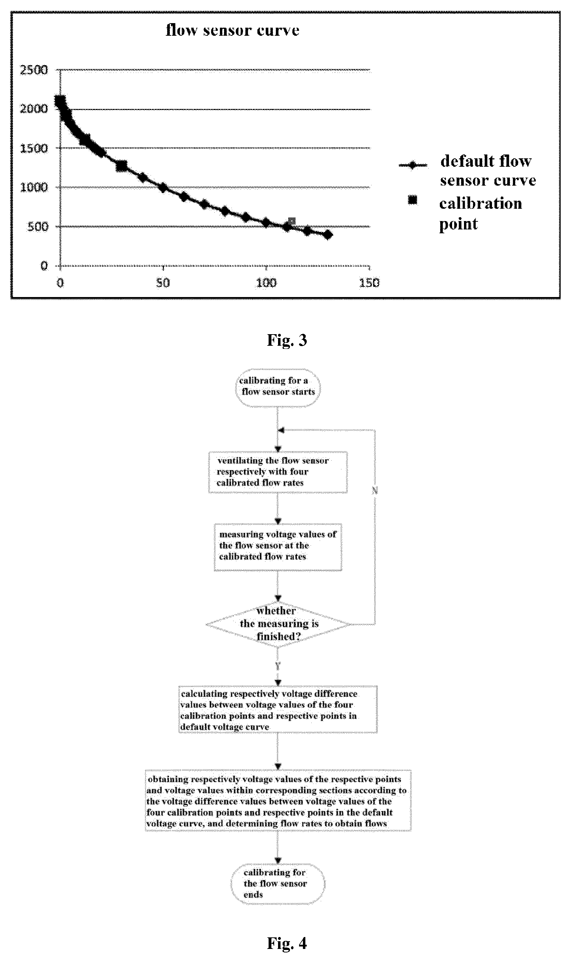

Fig. 2 is a flow chart for calibrating a default voltage curve according to one embodiment of the present invention. As shown in Fig. 2, a multi-point calibration, ranging from 0 L/min to 130 L/min, is performed on a flow sensor of each anesthesia machine among a plurality of anesthesia machines, the flow sensor is ventilated while a flow is monitored using a flowmeter, and a voltage value of the flow sensor corresponding to the flow is monitored. For example, when an airflow of 3 L/min is ventilated, a monitored voltage value is 1.998v, a flow and a corresponding voltage value are recorded. The flow is gradually increased within a range from 0 L/min to 5 L/min. As 0.5 L/min is increased every time, a flow and a corresponding voltage value are recorded. 11 pairs of acquired points are recorded within a range from 0 L/min to 5 L/min. The flow is gradually increased within a range from 5 L/min to 20 L/min, as 1 L/min is increased every time, a flow rate and a corresponding voltage value are recorded, and 15 pairs of acquired points are recorded within this range. Similarly, as 10 L/min is increased every time within a range from 20 L/min to 130 L/min, a flow rate and a corresponding voltage value are recorded. 11 pairs of acquired points are recorded within this range. 37 pairs of acquired points are recorded in total within a range from 0 L/min to 130 L/min. The 37 acquired points are connected to form a default voltage curve. A curve of a flow rate and a voltage of a flow sensor is shown in Fig. 3.

In one embodiment of the present invention, a range from 0 L/min to 130 L/min is divided into three sections and four calibration points are selected in the three sections. The three sections are respectively a first section ranging from 0 L/min to 5 L/min, a second section ranging from 5 L/min to 20 L/min and a third section ranging from 20 L/min to maximum of 130 L/min.

Fig. 4 is a flow chart for calibrating a flow of a flow sensor of each anesthesia machine according to one embodiment of the present invention. As shown in Fig. 4, four points are calibrated, the calibration method for each point is the same as the calibration method of the calibration points in the default curve. A first calibration point is a point that the flow rate is 0 L/min, a second calibration point is a point that the flow rate is an intermediate value between 0 L/min and 5 L/min, i.e. a point that the flow rate is 3 L/min, a third calibration point is a point that the flow rate is an intermediate value between 5 L/min and 20 L/min, i.e. a point that the flow rate is 12 L/min, and a forth calibration point is a point that the flow rate is an representative value between 30 L/min and 130 L/min, i.e. a point that the flow rate is 30 L/min. The four calibration points are respectively utilized to translate and correct the data in the present section to obtain all of the data points used by a real-time calculation. For example, in a default curve, the point that the flow rate is 0 L/min corresponds to a voltage value of 2.100v of the flow sensor, when a new flow sensor is calibrated, the point that the flow rate is 0 L/min corresponds to a voltage value of 2.098v of the flow sensor, then 2.100v is replaced with 2.098v; in the default curve, the point that the flow rate is 3 L/min corresponds to a voltage value of 1.998 v of the flow sensor, when a new flow sensor is calibrated, the point that the flow rate is 3 L/min corresponds to a voltage value of 1.980v of the flow sensor, then a difference value between the two voltage values is calculated as 0.020v, i.e. (1.998v-1.980v), 10 points ranging from 0.5 L/min to 5 L/min are corrected with the difference value, that is, the voltage values of the 10 points in the default curve all plus 0.020v as calibration values for the new flow sensor. Similarly, voltage values corresponding to flow rates ranging from 5 L/min to 20 L/min and ranging from 30 L/min to 130 L/min are corrected. The flow rates are obtained according to the voltage values and then the flow are obtained.

According to a method of the embodiments of the present invention, calibration points are determined within sectioned intervals and the flows of other acquired points are determined according to voltage difference values between default voltage curves and calibration points, therefore the degree of complexity of a calibration process is reduced, it is convenient for a user to use, meanwhile the accuracy of calibration is ensured.

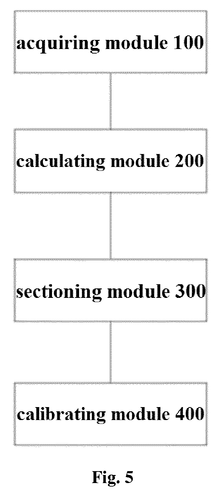

Fig. 5 is a structural block diagram of a flow calibration system for flow sensors of anesthesia machines according to one embodiment of the present invention. As shown in Fig. 5, the flow calibration system for flow sensors of anesthesia machines according to an embodiment of the present invention comprises an acquiring module 100, a calculating module 200, a sectioning module 300 and a calibrating module 400.

Wherein, the acquiring module 100 is configured for gradually incrementing flow rates of a plurality of anesthesia machines and acquiring voltage values of flow sensors when a preset condition is satisfied. Wherein the preset condition is that a flow rate of an anesthesia machine reaches an integral multiple of a preset flow rate.

The calculating module 200 is configured for calculating an average voltage value of each anesthesia machine at a plurality of sampling points and obtaining default voltage curves of flow sensors according to the average voltage value of each anesthesia machine at a plurality of sampling points.

The sectioning module 300 is configured for dividing the default curve into N sections, selecting N+1 calibration points in the N sections, and acquiring a voltage value of each calibration point, wherein N is a positive integer greater than 1.

In one embodiment of the present invention, N is three, i.e. the default curve is divided into three sections, and four points are calibrated in the three sections, and a first section is from a point that the flow rate is 0 to a point that the flow rate is 5 L/min, a second section is from a point that the flow rate is 5 L/min to a point that the flow rate is 20 L/min, and a third section is from a point that the flow rate is 20 L/min to a point that the flow rate is 130 L/min. The N+1 calibration points are four calibration points, wherein, the four calibration points are respectively a point that the flow rate is 0, a point that the flow rate is 3 L/min, a point that the flow rate is 12 L/min, and a point that the flow rate is 30 L/min.

In one embodiment of the present invention, preset flow rates in the three sections are different from each other. The preset flow rate in the first section is 0.5 L/min, the preset flow rate in the second section is 1 L/min, and the preset flow rate in the third section is 10 L/min.

The calibrating module 400 is configured for calculating a voltage difference value between the acquired voltage value of each calibration point and the default voltage curve, and accurately calibrating flows of anesthesia machines according to the voltage difference values.

In one embodiment of the present invention, a multi-point calibration is performed on a flow sensor of each anesthesia machine among a plurality of anesthesia machines within a range from 0 L/min to 130L/min by the acquiring module 100, and when a flow sensor is ventilated, a voltage value of the flow sensor is acquired. For example, as an airflow of 3 L/min is ventilated, a voltage value acquired by the acquiring module 100 is 1.998v, and a flow and a corresponding voltage value are recorded. The flow is gradually increased within a range from 0 L/min to 5 L/min, as 0.5 L/min is increased every time, a flow and a corresponding voltage value are recorded, 11 pairs of acquired points are recorded within the range from 0 L/min to 5 L/min. The flow is gradually increased within a range from 5 L/min to 20 L/min, as 1 L/min is increased every time, a flow rate and a corresponding voltage value are recorded, and 15 pairs of acquired points are recorded within this range. Similarly, when the acquiring module 100 increases a flow by 10 L/min every time within a range from 20 L/min to 130 L/min, a flow rate and a corresponding voltage value are acquired and recorded. 11 pairs of acquired points are recorded within this range. 37 pairs of acquired points are recorded in total within a range from 0 L/min to 130 L/min. The calculating module 200 connects the 37 acquired points to obtain a default voltage curve. A curve of a flow rate and a voltage of the flow sensor is shown in Fig. 3.

The sectioning module 300 is configured for dividing flow rates ranging from 0 L/min to 130 L/min into three sections and selecting four calibration points in the three sections, which are respectively a first section from 0 L/min to 5 L/min, a second section from 5 L/min to 20 L/min, and the third section from 20 L/min to maximum of 130 L/min.

In one embodiment of the present invention, the calibrating module 400 selects four calibration points in the three sections. A first calibration point is a point that the flow rate is 0 L/min, a second calibration point is a point that the flow rate is an intermediate value between 0 L/min and 5 L/min, i.e. a point that the flow rate is 3 L/min, a third calibration point is a point that the flow rate is an intermediate value between 5 L/min and 20 L/min, i.e. a point that the flow rate is 12 L/min, a forth calibration point is a point that the flow rate is an representative value between 30 L/min and 130 L/min, i.e. a point that the flow rate is 30 L/min. The four calibration points are utilized respectively to translate and correct the data of the present section in the three sections to obtain all of the data points used for a real-time calculation. For example, in a default curve, the point that the flow rate is 0 L/min corresponds to a voltage value of 2.100v of the flow sensor, as a new flow sensor is calibrated, the point that the flow rate is 0 L/min corresponds to a voltage value of 2.098v of the flow sensor, then 2.100 v is replaced with 2.098 v; in the default curve, the point that the flow rate is 3 L/min corresponds to a voltage value of 1.998v of the flow sensor, as a new flow sensor is calibrated, the point that the flow rate is 3 L/min corresponds to a voltage value of 1.980v of the flow sensor, a difference value between the two voltage values is calculated as 0.020v, i.e. (1.998v-1.980v), the difference value is used to correct the 10 points from 0.5 L/min to 5 L/min, that is, the voltage values of the 10 points in the default curve all plus 0.020v as calibration values of the new flow sensor. Similarly, the voltage values corresponding to flow rates ranging from 5 L/min to 20 L/min and ranging from 30 L/min to 130 L/min are corrected. The calibrating module 400 obtains flow rates according to the voltage values and then obtains flows of the anesthesia machines according to the flow rates and time and the like.

According to a system of the embodiments of the present invention, calibration points are determined within sectioned intervals and the flows of other acquired points are determined according to voltage difference values between default voltage curves and the voltage of the calibration points, therefore the degree of complexity of a calibration process is reduced, it is convenient for a user to use, meanwhile the accuracy of calibration is ensured.

Although the embodiments of the present invention have been shown and described above, it is to be understood that the above described embodiments are exemplary rather than limiting of the present invention. Those skilled in the art may make changes, alterations, substitution and modifications to the above described embodiments within the scope of the present invention without departing from the principle and gist of the present invention.

## Claims

1. A flow calibration method for flow sensors of anesthesia machines, comprising the following steps:
gradually incrementing flow rates of a plurality of anesthesia machines, and acquiring voltage values of the flow sensors when a preset condition is satisfied;
calculating an average voltage value of each anesthesia machine at a plurality of sampling points and obtaining default voltage curves of the flow sensors through the average voltage value of each anesthesia machine at the plurality of sampling points;
dividing the default voltage curves into N sections, selecting N+1 calibration points in the N sections, and acquiring a voltage value of each calibration point, wherein the N is a positive integer greater than 1; and
calculating a voltage difference value between the acquired voltage value of each calibration point and the default voltage curves, and accurately calibrating flows of the anesthesia machines according to the voltage difference values.

2. The flow calibration method for flow sensors of anesthesia machines of claim 1, wherein the N sections are three sections, and a first section is from a point that the flow rate is 0 L/min to a point that the flow rate is 5 L/min, a second section is from a point that the flow rate is 5 L/min to a point that the flow rate is 20 L/min, and a third section is from a point that the flow rate is 20 L/min to a point that the flow rate is 130 L/min.

3. The flow calibration method for flow sensors of anesthesia machines of claim 1, wherein the preset condition is that the flow rate of the anesthesia machines reaches an integral multiple of a preset flow rate.

4. The flow calibration method for flow sensors of anesthesia machines of claim 3, wherein the preset flow rates in the three sections are different from each other.

5. The flow calibration method for flow sensors of anesthesia machines of claim 1, wherein the N+1 calibration points are four calibration points, which are respectively a point that the flow rate is 0, a point that the flow rate is 3 L/min, a point that the flow rate is 12 L/min, and a point that the flow rate is 30 L/min.

6. A flow calibration system for flow sensors of anesthesia machines, comprising
an acquiring module configured for gradually incrementing flow rates of a plurality of anesthesia machines, and acquiring voltage values of the flow sensors when a preset condition is satisfied;
a calculating module configured for calculating an average voltage value of each anesthesia machine at a plurality of sampling points and obtaining default voltage curves of the flow sensors through the average voltage value of each anesthesia machine at the plurality of sampling points;
a sectioning module configured for dividing the default voltage curves into N sections, selecting N+1 calibration points in the N sections, and acquiring a voltage value of each calibration point, wherein the N is a positive integer greater than 1; and
a calibrating module configured for calculating a voltage difference value between the acquired voltage value of each calibration point and the default voltage curves, and accurately calibrating flows of the anesthesia machines according to the voltage difference values.

7. The flow calibration system for flow sensors of anesthesia machines of claim 6, wherein the N sections are three sections, and a first section is from a point that the flow rate is 0 L/min to a point that the flow rate is 5 L/min, a second section is from a point that the flow rate is 5 L/min to a point that the flow rate is 20 L/min, and a third section is from a point that the flow rate is 20 L/min to a point that the flow rate is 130 L/min.

8. The flow calibration system for flow sensors of anesthesia machines of claim 6, wherein the preset condition is that the flow rate of the anesthesia machines reaches an integral multiple of a preset flow rate.

9. The flow calibration system for flow sensors of anesthesia machines of claim 8, wherein the preset flow rates in the three sections are different from each other.

10. The flow calibration system for flow sensors of anesthesia machines of claim 6, wherein the N+1 calibration points are four calibration points, which are respectively a point that the flow rate is 0, a point that the flow rate is 3 L/min, a point that the flow rate is 12 L/min, and a point that the flow rate is 30 L/min.
